(19) Europäisches Patentamt
European Patent Office
Office européen des brevets

(11) EP 0 571 086 B1

(12) **EUROPEAN PATENT SPECIFICATION**

(45) Date of publication and mention of the grant of the patent:
**09.04.1997 Bulletin 1997/15**

(51) Int. Cl.$^6$: **A61K 7/06**, A61K 7/48

(21) Application number: **93303120.5**

(22) Date of filing: **21.04.1993**

(54) **Hair care composition**

Haarpflegemittel

Composition pour le soin des cheveux

(84) Designated Contracting States:
**AT BE CH DE DK ES FR GB GR IE IT LI NL PT SE**

(30) Priority: **22.04.1992 GB 9208652**

(43) Date of publication of application:
**24.11.1993 Bulletin 1993/47**

(73) Proprietors:
- **UNILEVER PLC**
  **London EC4P 4BQ (GB)**
  Designated Contracting States:
  **GB IE**
- **UNILEVER N.V.**
  **3013 AL Rotterdam (NL)**
  Designated Contracting States:
  **BE CH DE DK ES FR GR IT LI NL PT SE AT**

(72) Inventors:
- **Gough, Anthony David**
  **Eastham, Wirral, Merseyside L62 8HE (GB)**
- **Murray, Andrew Malcolm**
  **Parkgate, South Wirral, Cheshire L64 6TL (GB)**
- **Smith, Brian McDonald**
  **County Down B718 0HB, Northern Ireland (GB)**

(74) Representative: **Mulder, Cornelis Willem Reinier, Dr. et al**
**Unilever PLC,**
**Patent Division,**
**Colworth House**
**Sharnbrook, Bedford MK44 1LQ (GB)**

(56) References cited:
**EP-A- 0 309 052**
**EP-A- 0 367 939**
**EP-A- 0 409 502**
**EP-A- 0 409 504**
**FR-A- 2 356 627**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 571 086 B1

## Description

This invention relates to hair care compositions, more particularly to hair conditioning compositions.

A class of conditioning agents which are known for inclusion in compositions for treating the hair to provide one or more tactile and/or visual benefits such as smoothness, softness, shine, non-flyaway, ease of wet and/or dry combing, are the quaternary ammonium salts represented by the following formula I:

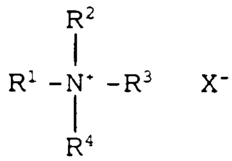

(I)

wherein each of at least one of $R^1$ and $R^2$ independently represents a long chain alkyl or alkenyl group containing from about 8 to about 23 carbon atoms; $R^3$ and $R^4$ (and $R^1$ or $R^2$ if not a group as defined above) are each independently an alkyl or hydroxyalkyl group containing from 1 to 4 carbon atoms, or a benzyl group; and $X^-$ is a water soluble anion, e.g. chloride. Examples of quaternary ammonium salts of this class include cetyltrimethylammonium chloride and ditallowdimethylammonium chloride.

This class of conditioning agents is favoured over many other types of conditioning agent because of their superior hair conditioning properties.

There are however several problems associated with these cationic hair conditioning agents, in particular their aqueous toxicity and low biodegradability, which makes these compounds environmentally unfriendly, as well as the tendency of certain compounds of this class, e.g. cetyltrimethylammonium chloride, to be irritant to the eyes. These characteristics make this class of conditioning agents unattractive for consumer use.

In a different field, namely that of fabric conditioning agents, another class of quaternary ammonium salts which are known to possess good fibre softening properties, are those compounds represented generally by the following formula II:

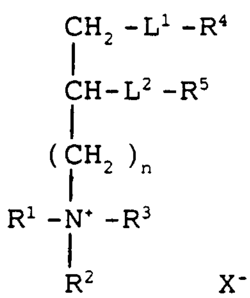

(II)

wherein $R^1$, $R^2$ and $R^3$ are each independently an alkyl or hydroxyalkyl group containing from 1 to 4 carbon atoms, or a benzyl group; $R^4$ and $R^5$ each independently represents a long chain alkyl or alkenyl group containing from 8 to 23 carbon atoms; $L^1$ and $L^2$ each independently represents a group of the formula:

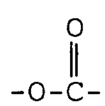 , 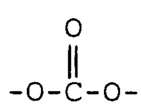 or 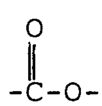 ;

n is an integer from 0 to about 5; and $X^-$ is a water soluble anion, e.g. chloride.

These di-ester-linked quaternary ammonium compounds and their methods of preparation are fully described in for example GB 1567947 (Unilever), the disclosure of which is incorporated herein by reference. The use of compounds of this class as fabric softening agents is further described in for example EP-A-409 502 (Unilever), EP-A-409 504 (Unilever) and EP-A-420 465 (Unilever).

Surprisingly, it has now been found that the above mentioned class of ester-linked quaternary ammonium compounds, when applied to the hair, result in excellent conditioning performance, especially with respect to the attributes

ease of wet combing and non-flyaway. Furthermore, as well as these compounds being much more readily biodegradable, less toxic in the natural environment and less irritant to the eyes of a user, they are significantly better hair conditioning agents than the known mono-alkyl and di-alkyl non-ester-linked groups of compounds mentioned above.

This finding is even more surprising, given that also known to have good hair conditioning properties are the mono-ester-linked quaternary ammonium compounds corresponding to the above formula II, but having only one ester-linked long chain group, but which compounds still exhibit unacceptably high aqueous toxicity.

Accordingly, in a first aspect the present invention provides a method of conditioning hair comprising applying thereto a composition comprising an ester-linked quaternary ammonium compound represented by the following formula III:

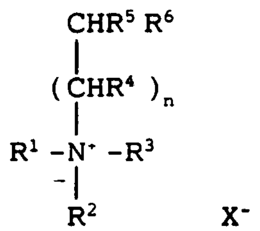

(III)

wherein n is an integer of from 1 to about 8, preferably 1 to about 7, more preferably 1 to about 5 or 6; $R^1$, $R^2$ and $R^3$ are each independently an alkyl or hydroxyalkyl group containing from about 1 to about 4 carbon atoms, or a benzyl group; each $R^4$ and each of $R^5$ and $R^6$ are independently selected from -H, -OH,

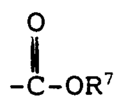 , 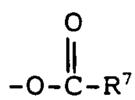 or 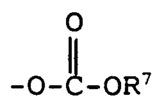 ,

wherein $R^7$ is a linear or branched long chain alkyl or alkenyl group containing from about 8 to about 28 carbon atoms; and $X^-$ is a water soluble anion;
with the proviso that at least two of the groups selected from any $R^4$, $R^5$ and $R^6$ are other than -H or -OH.

The present invention will now be described in detail.

The primary hair conditioning agent in the hair care compositions of the present invention is that defined by formula III above. Such compounds may be used either singly or in combination, e.g. mixtures, as the primary hair conditioning agent.

Compounds of this formula suitable for use in the hair care compositions of the present invention include those disclosed for example in GB 1567947 and US 4137180, the disclosures of both of which are incorporated herein by reference.

Preferred compounds for use in the invention are those of the following formula IV:

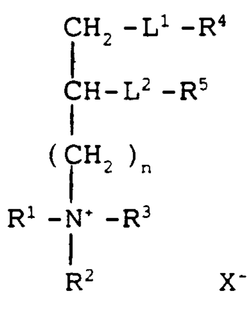

(IV)

wherein $R^1$, $R^2$ and $R^3$ are each independently an alkyl or hydroxyalkyl group containing from about 1 to about 4 carbon atoms, or a benzyl group; $R^4$ and $R^5$ each independently represents a linear or branched long chain alkyl or alkenyl group containing from about 8 to about 28 carbon atoms; $L^1$ and $L^2$ each independently represents a group of the formula:

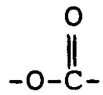 , 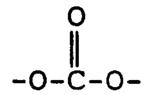 or 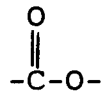 ;

n is an integer from 0 to about 5; and $X^-$ is a water soluble anion.

Particularly preferred compounds for use in the invention include those of the above formula IV, wherein $R^1$, $R^2$ and $R^3$ are all methyl, $R^4$ and $R^5$ are each independently an alkyl group having from 14 to 18 carbon atoms, preferably having an average chain length of 18 carbon atoms, $L^1$ and $L^2$ are as defined above, n is 1 and the counterion $X^-$ is chloride.

The preferred level of the ester-linked quaternary ammonium compound incorporated as the conditioning agent in the hair care compositions of the invention is from about 0.01 to about 20% by weight of the composition, more preferably from 0.05 to 10%, even more preferably 0.1 to 5%.

The hair care compositions of the present invention may further comprise one or more optional ingredients which are normally found in hair care compositions. The compositions of the invention may therefore not only take the form of hair conditioning compositions as such, but may also take the form of conditioning shampoos or hair styling compositions or the like. Such compositions in accordance with the invention may be provided in any suitable physical form, for example pourable liquids, lotions, mousses, sprays, etc, as are well known in the art.

Generally, it is preferred that any additional ingredients do not substantially affect the biodegradability of the composition. Therefore it is preferred to minimise the level of non-biodegradable or less biodegradable additional materials in the compositions of the invention.

One preferred optional component which may be included in the hair care compositions of the invention is a fatty alcohol or fatty acid, or derivative thereof, or a mixture of any of these, having a chain length of from about 8 to about 28 carbon atoms, more preferably from about 12 to about 18 carbon atoms. These materials may be predominantly linear or may be branched.

Such fatty material(s) may be present in the compositions of the invention in a total amount of from about 0.001 to 20% by weight, more preferably 0.01 to 10%, even more preferably 0.01 to 5% yet more preferably 0.1 to 1%. An especially preferred amount of the fatty material, if present, is up to about 0.5% by weight, since such amounts help to render the compositions smooth textured and non-lumpy.

Where it is desired to formulate a hair care composition of the invention which not only has conditioning properties but also has detergent properties, i.e. a shampoo, then one or more surfactants may be included, preferably selected from nonionic, amphoteric and zwitterionic surfactants.

Nonionic surfactants suitable for use in compositions of the invention include condensation products of aliphatic ($C_8$-$C_{18}$) primary or secondary linear or branched chain alcohols or phenols with alkylene oxides, usually ethylene oxide, and generally having from 6 to 30 ethylene oxide groups.

Other suitable nonionics include mono- or di-alkyl alkanolamides. Examples include coco mono- or di-ethanolamide and coco mono-isopropanolamide.

Further suitable nonionic surfactants are the alkyl polyglycosides (APG's). Typically, the APG is one which comprises an alkyl group connected (optionally via a bridging group) to a block of one or more glycosyl groups. Preferred APG's are described by the following formula:

$$RO\text{-}(G)_n$$

wherein R is a branched or straight chain alkyl group which may be saturated or unsaturated and G is a saccharide group.

R may represent a mean alkyl chain length of from about $C_5$ to about $C_{20}$. Preferably, R represents a mean alkyl chain length of from about $C_8$ to about $C_{12}$. Most preferably the value of R lies between about 9.5 and about 10.5. G may be selected from $C_5$ or $C_6$ monosaccharide residues or mixtures of $C_5$ and $C_6$ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies in the range of from about 1.1 to about 2. Most preferably the value of n lies in the range of from about 1.3 to about 1.5.

Suitable alkyl polyglycosides for use in the invention are commercially available and include for example those materials identified as: Oramix NS10 ex Seppic; and APG225, APG300, APG350, APG550 and APG600 ex Henkel.

Amphoteric and zwitterionic surfactants suitable for use in compositions of the invention may include alkyl amine oxides, alkyl betaines, alkyl amidopropyl betaines, alkyl sulphobetaines (sultaines), alkyl glycinates, alkyl carboxyglycinates, alkyl amphopropionates, alkylamphoglycinates alkyl amidopropyl hydroxysultaines, acyl taurates and acyl glutamates, wherein the alkyl and acyl groups have from 8 to 19 carbon atoms. Examples include lauryl amine oxide, cocodimethyl sulphopropyl betaine and preferably lauryl betaine, cocamidopropyl betaine and sodium cocamphopropionate.

Further surfactants which may be suitable for use in shampoos in accordance with the invention include one or more anionic surfactants instead of or in addition to any of those surfactants mentioned above.

Suitable anionic surfactants are the alkyl sulphates, alkyl ether sulphates, alkaryl sulphonates, alkanoyl isethionates, alkyl succinates, alkyl sulphosuccinates, N-alkoyl sarcosinates, alkyl phosphates, alkyl ether phosphates, alkyl ether carboxylates, and alpha-olefin sulphonates, especially their sodium, magnesium, ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from one to 10 ethylene oxide or propylene oxide units per molecule, and preferably contain 2 to 3 ethylene oxide units per molecule.

Examples of suitable anionic surfactants include sodium oleyl succinate, ammonium lauryl sulphosuccinate, ammonium lauryl sulphate, sodium dodecylbenzene sulphonate, triethanolamine dodecylbenzene sulphonate, sodium coroyl isethionate, sodium lauroyl isethionate and sodium N-lauryl sarcosinate. The most preferred anionic surfactants are sodium lauryl sulphate, triethanolamine lauryl sulphate, triethanolamine monolauryl phosphate, sodium lauryl ether sulphate 1EO, 2EO and 3EO, ammonium lauryl sulphate and ammonium lauryl ether sulphate 1EO, 2EO and 3EO.

The surfactant(s) may be present in the hair care composition in a total amount of from about 1 to 70% by weight, preferably from 2 to 40% by weight, more preferably from 5 to 30% by weight.

The hair care compositions of the present invention may, in addition to the main ester-linked quaternary ammonium salt conditioning agent, contain one or more additional conditioning agents, as are well known in the art. Such additional conditioning agents may include cationic surfactants, cationic polymers, volatile or non-volatile silicones and other materials which are known in the art as having desirable hair conditioning properties.

The compositions of the invention may optionally include a deposition polymer to aid deposition of the conditioning agent(s) onto the hair. A suitable deposition polymer is a cationic derivative of guar gum.

Suitable cationic guar gum derivatives are those given the CTFA designation guar hydroxypropyl trimonium chloride, available commercially for example as JAGUAR C13S, which has a low degree of substitution of the cationic groups and a high viscosity. Other suitable materials include those known as JAGUAR C15, having a moderate degree of substitution and a low viscosity, JAGUAR C17 (high degree of substitution, high viscosity) and JAGUAR C16 (which is a hydroxypropylated cationic guar derivative containing a low level of substituent groups as well as cationic quaternary ammonium groups). Also suitable is JAGUAR C162 (which is a high transparency, medium viscosity guar derivative having a low degree of substitution).

As further optional components for inclusion in the compositions of the invention, in addition to water, the following may be mentioned: pH adjusting agents, viscosity modifiers, pearlescers, opacifiers, suspending agents, preservatives, perfumes, colouring agents, dyes, proteins, herb and plant extracts, antidandruff agents, polyols and other moisturising agents.

Embodiments of the present invention will now be further illustrated by reference to the following examples. All amounts given are in % by weight, unless otherwise stated.

EXAMPLES

Examples 1 to 6

Hair conditioning preparations were prepared by mixing the following ingredients in the amounts stated.

| Ingredient | Example | | | | | |
|---|---|---|---|---|---|---|
| | 1 | 2 | 3 | 4 | 5 | 6 |
| DTTMAPC[(1)] | 1.0 | 1.0 | 1.0 | 1.0 | 1.0 | 1.0 |
| Laurex CS[(2)] | - | - | 0.1 | 0.25 | - | - |
| Stearic acid | - | - | - | - | 0.1 | 0.25 |
| Natrosol[(3)] | - | 1.2 | 1.2 | 1.2 | 1.2 | 1.2 |
| Demineralised water | -----------to 100----------- | | | | | |

[(1)] 1,2-ditallowoxytrimethylammonium propane chloride
[(2)] Cetostearyl alcohol
[(3)] Hydroxyethyl cellulose

Examples 7 and 8

Hair shampoo compositions were prepared by mixing the following ingredients in the amounts stated. The DTTMAPC and APG (Oramix NS-10) were mixed together first, followed by addition of the remaining ingredients.

| Ingredient | Example | |
|---|---|---|
| | 7 | 8 |
| Oramix NS-10[(4)] | 16.0 | 16.0 |
| Cocoamido propyl betaine | 4.0 | - |
| PEG 6000 distearate | 2.0 | 2.0 |
| DTTMAPC | 1.0 | 1.0 |
| Perfume, colouring | qs | qs |
| Demineralised water | -----to 100---- | |

[(4)] Decyl glucoside, ex Seppic.

The superior hair conditioning properties of the ester-linked quaternary ammonium salts of the present invention, as compared with a standard non-conditioning shampoo formulation and also with monoalkyl quaternary ammonium salt conditioning agents of the prior art, were demonstrated by means of the following Comparative Examples.

Comparative Example 1

The hair conditioning composition of Example 1 above was tested against Timotei (trade mark) shampoo, a non-conditioning shampoo formulation, by the following procedure.

Six 8 gram (20cm) hair switches were shampooed twice using 0.5g of shampoo each time, for a 30 second wash time, then rinsed with water and dried. Three of the switches were then treated with the test conditioner for 30 seconds, left to stand for 60 seconds, followed by rinsing for 30 seconds, before combing and drying.

Six panellists then assessed the ease of dry combing, lack of flyaway and smoothness, comparing the test switches with the control switches treated with Timotei shampoo alone, in a paired comparison test. The results are shown in Table 1 below.

Table 1

| Hair attribute | Voting split (maximum 36) | |
|---|---|---|
| | Timotei Shampoo | Timotei Shampoo and Example 1 Conditioner |
| Ease of dry combing | 0 | 36 |
| Non-flyaway | 0 | 36 |
| Smoothness | 6 | 30 |

Comparative Example 2

The hair conditioning composition of Example 1 above was tested against a conditioning composition comprising 1% by weight cetyltrimethylammonium chloride, 2% by weight Laurex CS (cetostearyl alcohol) and the balance water, by the following procedure.

Six 8 gram (20cm) hair switches were shampooed with Timotei shampoo twice using 0.5g of shampoo each time, for a 30 second wash time, then rinsed with water and dried. Three of the switches were then treated with 1g of the test

conditioner for 30 seconds, left to stand for 60 seconds, followed by rinsing for 30 seconds, before combing and drying. The other three switches were likewise treated with the control 1% CTAC/2% Laurex CS conditioner.

Twelve panellists then assessed the ease of dry combing, lack of flyaway and smoothness, comparing test switches with control switches, in a paired comparison test. The results are shown in Table 2 below.

Table 2

| Hair attribute | Voting split (maximum 72) | |
|---|---|---|
| | Timotei Shampoo and Example 1 Conditioner | Timotei Shampoo and 1% CTAC/2% Laurex Conditioner |
| Ease of dry combing | 29 | 43 |
| Non-flyaway | 56* | 16 |
| Smoothness | 40 | 32 |

*95% significance

Comparative Example 3

The hair conditioning composition of Example 5 above was tested against a conventional UK commercially available conditioning formulation (Creme Silk (trade mark), ex Elida Gibbs) for ease of wet combing, by the following procedure.

A hair switch (8g) was washed with a surfactant solution (12% sodium lauryl ether sulphate 2EO in water), this solution being referred to hereafter as the surfactant base: 0.5g of the surfactant base was applied to the wetted hair, the switch lathered for 30 seconds and after leaving for a further 10 seconds, the hair was rinsed with water for 30 seconds. After removing excess water, the hair was combed until free of tangles with a comb which was linked to an instrument which measured the total combing time (TCT), as is known in the art. The TCT value after treatment with the surfactant base is T1. The hair switch was then treated with 0.5ml of Timotei shampoo, lathered for 30 seconds and after leaving for a further 10 seconds, the hair was rinsed with water for 30 seconds. The hair switch was then treated with 1g of Example conditioner 5 which was massaged into the hair for 30 seconds. After leaving for 60 seconds the hair switch was rinsed with water for 30 seconds. After removing excess water, the switch was again combed until free of tangles to give a TCT value T2. The combing time after treatment with the test hair conditioner expressed as a percentage of that after treatment with the surfactant base is

$$\frac{T2}{T1} \times 100.$$

The procedure was carried out on two other hair switches and the average of the percentage values for the three switches was taken as the wet combing value for the test product. Thus, the more effective the product at conditioning the hair, the lower the wet combing value. A different set of three switches was used for each test product. The results are given in Table 3 below.

Table 3

| Formulation | % Total Combing Time |
|---|---|
| Creme Silk Conditioner (as sold in the UK) | 21.4 (sd 1.9) |
| Example Conditioner 5 | 15.2 (sd 4.0) |

Comparative Example 4

The dry hair conditioning properties of the conditioning composition of Example 6 were compared with those of the

same control conditioning formulation used in Comparative Example 2 above, using the same procedure as in that Comparative Example. The results of the paired comparison test are given in Table 4 below.

Table 4

| Hair attribute | Voting split (maximum 72) | |
|---|---|---|
| | Timotei Shampoo and Example 6 Conditioner | Timotei Shampoo and 1% CTAC/2% Laurex Conditioner |
| Ease of dry combing | 54* | 18 |
| Non-flyaway | 62* | 10 |
| Smoothness | 48* | 24 |

*95% significance

With this test formulation significantly better dry combing and smoothness properties are obtained in addition to superior antistatic benefits.

<u>Comparative Example 5</u>

The conditioning shampoo composition of Example 7 above was tested against the same formulation in which the DTTMAPC conditioning agent of the invention had been omitted (base shampoo), using the same procedure as in Comparative Example 1. The results of the paired comparison test are given in Table 5 below.

Table 5

| Hair attribute | Voting split (maximum 72) | |
|---|---|---|
| | Shampoo Composition 7 | Base Shampoo |
| Ease of dry combing | 64* | 8 |
| Non-flyaway | 67* | 5 |
| Smoothness | 60* | 12 |

*95% significance

## Claims

**1.** A method of conditioning hair comprising applying thereto a composition comprising an ester-linked quaternary ammonium compound represented by the formula:

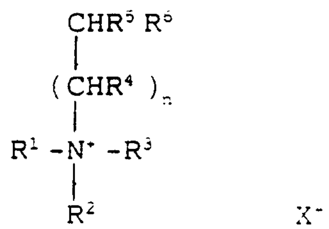

wherein n is an integer of from 1 to 8; $R^1$, $R^2$ and $R^3$ are each independently an alkyl or hydroxyalkyl group containing from 1 to 4 carbon atoms, or a benzyl group; each $R^4$ and each of $R^5$ and $R^6$ are independently selected from -H, -OH,

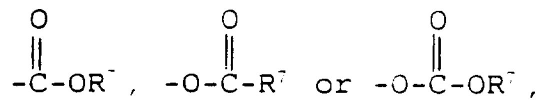

wherein $R^7$ is a linear or branched long chain alkyl or alkenyl group containing from 8 to 28 carbon atoms; and $X^-$ is a water soluble anion; with the proviso that at least two of the groups selected from any $R^4$, $R^5$ and $R^6$ are other than -H or -OH.

**2.** A method according to claim 1 wherein the ester-linked quaternary ammonium compound is of the formula:

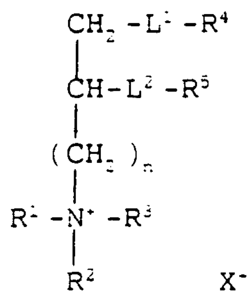

wherein $R^1$, $R^2$ and $R^3$ are each independently an alkyl or hydroxyalkyl group containing from 1 to 4 carbon atoms, or a benzyl group; $R^4$ and $R^5$ each independently represents a linear or branched long chain alkyl or alkenyl group containing from 8 to 28 carbon atoms; $L^1$ and $L^2$ each independently represents a group of the formula:

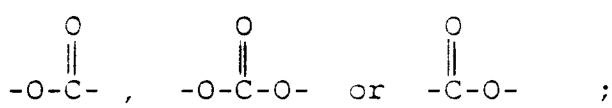

n is an integer from 0 to 5; and $X^-$ is a water soluble anion.

**3.** A method according to claim 2, therein in the formula $R^1$, $R^2$ and $R^3$ are all methyl, $R^4$ and $R^5$ are each independently an alkyl group having from 14 to 18 carbon atoms, n is 1 and the counterion $X^-$ is chloride.

**4.** A method according to any one of claims 1 to 3, wherein the ester-linked quaternary ammonium compound is present in an amount of from 0.01 to 20% by weight of the composition.

**5.** A method according to any preceding claim, wherein the composition further comprises a fatty alcohol or fatty acid, or derivative thereof, or a mixture of any of these, having a chain length of from 8 to 28 carbon atoms.

**6.** A method according to claim 5, wherein the fatty material is present in a total amount of from 0.001 to 20% by weight.

**7.** A method according to claim 6, wherein the fatty material is present in an amount of up to 0.5% by weight.

**8.** A method according to any preceding claim, wherein the composition further comprises one or more surfactants selected from nonionic, amphoteric and zwitterionic surfactants.

**9.** A method according to any preceding claim, wherein the composition further comprises a deposition polymer.

**10.** Use as a hair conditioning agent of an ester-linked quaternary ammonium compound represented by the formula:

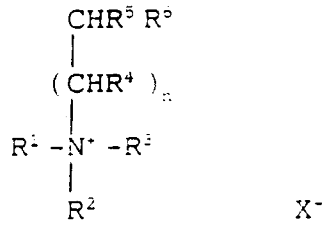

wherein n is an integer of from 1 to 8; $R^1$, $R^2$ and $R^3$ are each independently, an alkyl or hydroxyalkyl group containing from 1 to 4 carbon atoms, or a benzyl group; each $R^4$ and each of $R^5$ and $R^6$ are independently selected from -H, -OH,

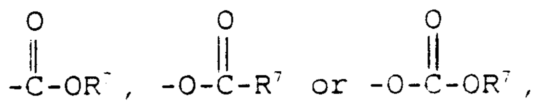

wherein $R^7$ is a linear or branched long chain alkyl or alkenyl group containing from 8 to 28 carbon atoms; and $X^-$ is a water soluble anion;
with the proviso that at least two of the groups selected from any $R^4$, $R^5$ and $R^6$ are other than -H or -OH.

**11.** A hair care composition comprising:

(i) an ester-linked quaternary ammonium compound represented by the formula:

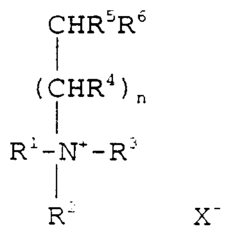

wherein n is an integer of from 1 to 8; $R^1$, $R^2$ and $R^3$ are each independently an alkyl or hydroxyalkyl group containing from 1 to 4 carbon atoms, or a benzyl group; each $R^4$ and each of $R^5$ and $R^6$ are independently selected from -H, -OH,

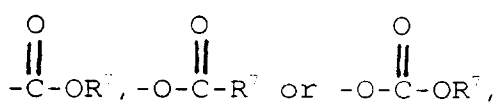

wherein $R^7$ is a linear or branched long chain alkyl or alkenyl group containing from 8 to 28 carbon atoms; and $X^-$ is a water soluble anion;
with the proviso that at least two of the groups selected from any $R^4$, $R^5$ and $R^6$ are other than -H or -OH; and

(ii) from about 1 to 70% by weight of a $C_5$-$C_{20}$ alkyl polyglycoside.

**12.** Use, in the preparation of a composition for conditioning hair, of

an ester-linked quaternary ammonium compound represented by the formula:

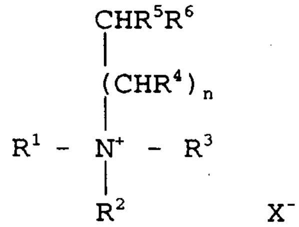

wherein n is an integer of from 1 to 8; $R^1$, $R^2$ and $R^3$ are each independently an alkyl or hydroxyalkyl group containing from 1 to 4 carbon atoms, or a benzyl group; each $R^4$ and each of $R^5$ and $R^6$ are independently selected from -H, -OH,

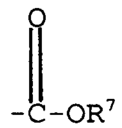, 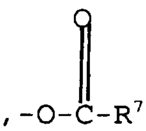 or 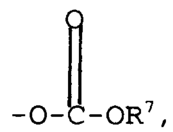,

wherein $R^7$ is a linear or branched long chain alkyl or alkenyl group containing from 8 to 28 carbon atoms; and $X^-$ is
a water soluble anion; with the proviso that at least two of the groups selected from any $R^4$, $R^5$ and $R^6$ are other than -H or -OH.

## Patentansprüche

1. Verfahren zur Konditionierung von Haar durch Applizieren einer Zusammensetzung, die eine esterverknüpfte quaternäre Ammoniumverbindung der folgenden Formel umfaßt:

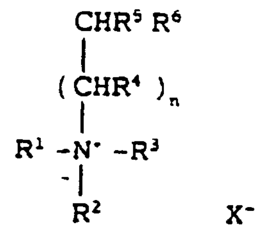

worin n eine ganze Zahl von 1 bis 8 ist, $R^1$, $R^2$ und $R^3$ jeweils unabhängig voneinander für eine Alkyl- oder Hydroxyalkylgruppe mit 1 bis 4 Kohlenstoffatom(en) oder eine Benzylgruppe stehen, jeder Rest $R^4$ und jeder Rest $R^5$ und $R^6$ unabhängig voneinander unter -H, -OH,

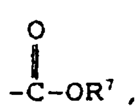, 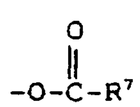 oder 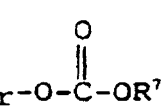

ausgewählt sind, wobei $R^7$ für eine lineare oder verzweigte langkettige Alkyl- oder Alkenylgruppe mit 8 bis 28 Kohlenstoffatomen steht, und $X^-$ ein wasserlösliches Anion ist, wobei gilt. daß mindestens zwei der Gruppen $R^4$, $R^5$ oder $R^6$ von -H oder -OH verschieden sind.

2. Verfahren nach Anspruch 1, wobei die esterverknüpfte quaternäre Ammoniumverbindung der folgenden Formel

entspricht:

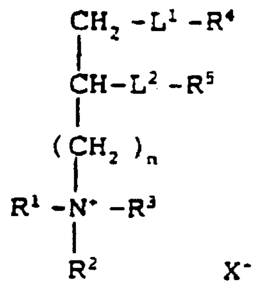

worin $R^1$, $R^2$ und $R^3$ jeweils unabhängig voneinander für eine Alkyl- oder Hydroxyalkylgruppe mit 1 bis 4 Kohlenstoffatom(en) oder eine Benzylgruppe stehen, $R^4$ und $R^5$ jeweils unabhängig voneinander eine lineare oder verzweigte langkettige Alkyl- oder Alkenylgruppe mit 8 bis 28 Kohlenstoffatomen bedeuten, $L^1$ und $L^2$ jeweils unabhängig voneinander für eine Gruppe der Formel:

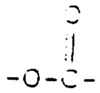 , 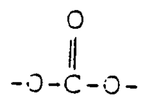 oder 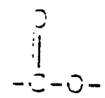

stehen, n eine ganze Zahl von 0 bis 5 ist und $X^-$ einem wasserlöslichen Anion entspricht.

**3.** Verfahren nach Anspruch 2, wobei in der Formel $R^1$, $R^2$ und $R^3$ alle für Methyl stehen, $R^4$ und $R^5$ jeweils unabhängig voneinander eine Alkylgruppe mit 14 bis 18 Kohlenstoffatomen bedeuten, n 1 ist und das Gegenion $X^-$Chlorid entspricht.

**4.** Verfahren nach einem der Ansprüche 1 bis 3, wobei die esterverknüpfte quaternäre Ammoniumverbindung in einer Menge von 0,01 bis 20 Gew.-% der Zusammensetzung vorhanden ist.

**5.** Verfahren nach einem der vorhergehenden Ansprüche, wobei die Zusammensetzung des weiteren einen Fettalkohol oder eine Fettsäure oder ein Derivat hiervon oder ein Gemisch aus einem beliebigen dieser Komponenten mit einer mittleren Kettenlänge von 8 bis 28 Kohlenstoffatomen umfaßt.

**6.** Verfahren nach Anspruch 5, wobei das Fettmaterial in einer Gesamtmenge von 0,001 bis 20 Gew.-% vorhanden ist.

**7.** Verfahren nach Anspruch 6, wobei das Fettmaterial in einer Menge von bis zu 0,5 Gew.-% vorhanden ist.

**8.** Verfahren nach einem der vorhergehenden Ansprüche, wobei die Zusammensetzung des weiteren ein oder mehrere unter nichtionischen, amphoteren und zwitterionischen grenzflächenaktiven Mitteln ausgewählte grenzflächenaktive Mittel umfaßt.

**9.** Verfahren nach einem der vorhergehenden Ansprüche, wobei die Zusammensetzung des weiteren ein Abscheidungspolymer umfaßt.

**10.** Verwendung einer esterverknüpften quaternären Ammoniumverbindung der Formel:

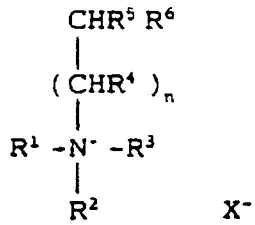

worin n eine ganze Zahl von 1 bis 8 ist, $R^1$, $R^2$ und $R^3$ jeweils unabhängig voneinander für eine Alkyl- oder Hydroxyalkylgruppe mit 1 bis 4 Kohlenstoffatom(en) oder eine Benzylgruppe stehen, jeder Rest $R^4$ und jeder Rest $R^5$ und $R^6$ unabhängig voneinander unter -H, -OH,

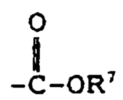 , 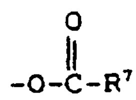 oder 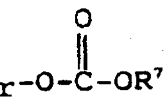

ausgewählt sind, wobei $R^7$ für eine lineare oder verzweigte langkettige Alkyl- oder Alkenylgruppe mit 8 bis 28 Kohlenstoffatomen steht, und $X^-$ ein wasserlösliches Anion ist, wobei gilt, daß mindestens zwei der Gruppen $R^4$, $R^5$ oder $R^6$ von -H oder -OH verschieden sind, als Haarkonditioniermittel.

**11.** Haarpflegezusammensetzung, die die folgenden Bestandteile umfaßt:

(i) eine esterverknüpfte quaternäre Ammoniumverbindung der Formel:

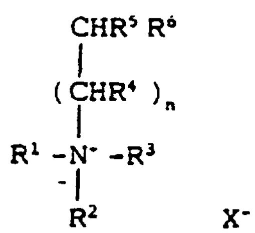

worin n eine ganze Zahl von 1 bis 8 ist, $R^1$, $R^2$ und $R^3$ jeweils unabhängig voneinander für eine Alkyl- oder Hydroxyalkylgruppe mit 1 bis 4 Kohlenstoffatom(en) oder eine Benzylgruppe stehen, jeder Rest $R^4$ und jeder Rest $R^5$ und $R^6$ unabhängig voneinander unter -H, -OH,

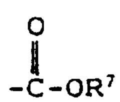 , 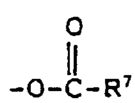 oder 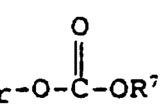

ausgewählt sind, wobei $R^7$ für eine lineare oder verzweigte langkettige Alkyl- oder Alkenylgruppe mit 8 bis 28 Kohlenstoffatomen steht, und $X^-$ ein wasserlösliches Anion ist, wobei gilt, daß mindestens zwei der Gruppen $R^4$, $R^5$ oder $R^6$ von -H oder -OH verschieden sind, und

(ii) etwa 1 bis 70 Gew.-% eines $C_5$-$C_{20}$-Alkylpolyglycosids.

**12.** Verwendung einer esterverknüpften quaternären Ammoniumverbindung der Formel:

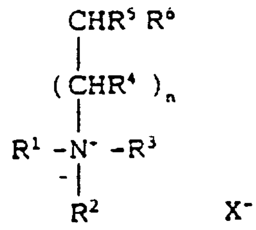

worin n eine ganze Zahl von 1 bis 8 ist, $R^1$, $R^2$ und $R^3$ jeweils unabhängig voneinander für eine Alkyl- oder Hydroxyalkylgruppe mit 1 bis 4 Kohlenstoffatom(en) oder eine Benzylgruppe stehen, jeder Rest $R^4$ und jeder Rest $R^5$ und $R^6$ unabhängig voneinander unter -H, -OH,

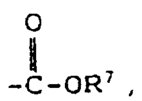 , 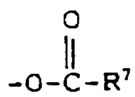 oder 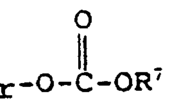

ausgewählt sind, wobei $R^7$ für eine lineare oder verzweigte langkettige Alkyl- oder Alkenylgruppe mit 8 bis 28 Kohlenstoffatomen steht, und $X^-$ ein wasserlösliches Anion ist, wobei gilt, daß mindestens zwei der Gruppen $R^4$, $R^5$ oder $R^6$ von -H oder -OH verschieden sind, bei der Herstellung einer Zusammensetzung zur Konditionierung von Haar.

## Revendications

1. Un procédé destiné au conditionnement des cheveux comprenant l'étape consistant à appliquer sur ceux-ci une composition comprenant un composé d'ammonium quaternaire lié par un ester représenté par la formule suivante :

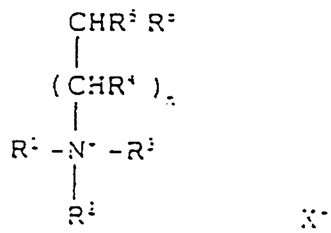

où n est un entier compris entre 1 et 8 ; $R^1$, $R^2$ et $R^3$ sont chacun indépendamment un groupe alkyle ou hydroxyalkyle comprenant 1 à 4 atome(s) de carbone ou un groupe benzyle ; chaque $R^4$ et chacun de $R^5$ et $R^6$ sont chacun indépendamment sélectionnés parmi -H, -OH,

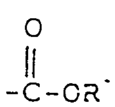 , 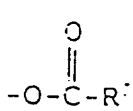 or 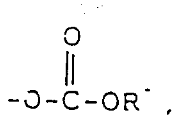 ,

où $R^7$ est un groupe alkyle ou alkényle à chaîne longue linéaire ou ramifiée comprenant 8 à 28 atomes de carbone : et $X^-$ est un anion soluble dans l'eau : sous réserve qu'au moins deux des groupes sélectionnés parmi $R^4$, $R^5$ et $R^6$ soient autres que -H ou -OH.

2. Un procédé selon la Revendication 1, dans lequel le composé d'ammonium quaternaire lié par un ester présente la formule suivante :

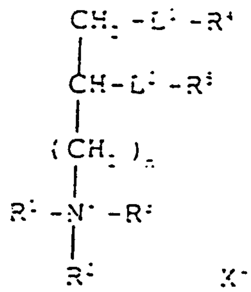

où $R^1$, $R^2$ et $R^3$ sont chacun indépendamment un groupe alkyle ou hydroxyalkyle comprenant 1 à 4 atome(s) de carbone ou un groupe benzyle : $R^4$ et $R^5$ représentant chacun indépendamment un groupe alkyle ou alkényle à chaîne longue linéaire ou ramifiée comprenant 8 à 28 atomes de carbone ; $L^1$ et $L^2$ représentent chacun indépendamment un groupe de la formule suivante :

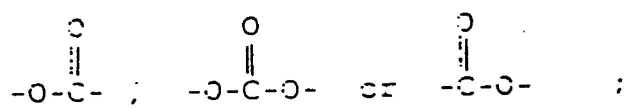

n est un entier compris entre 0 et 5 ; et $X^-$ est un anion soluble dans l'eau.

**3.** Un procédé selon la Revendication 2, dans lequel dans la formule $R^1$, $R^2$ et $R^3$ sont tous du méthyle, $R^4$ et $R^5$ représentant chacun indépendamment un groupe alkyle comprenant 14 à 18 atomes de carbone, n est égal à 1 et le contre-ion $X^-$ est du chlorure.

**4.** Un procédé selon l'une quelconque des Revendications 1 à 3, dans lequel le composé d'ammonium quaternaire lié par un ester est présent dans une proportion comprise entre 0,01 et 20 % en masse de la composition.

**5.** Un procédé selon l'une quelconque des Revendications précédentes, dans lequel la composition comprend en outre un alcool gras ou un acide gras, ou un dérivé de ceux-ci, ou un mélange de l'un quelconque de ceux-ci, présentant une longueur de chaîne comprise entre 8 et 28 atomes de carbone.

**6.** Un procédé selon la Revendication 5, dans lequel la matière grasse est présente dans une proportion totale comprise entre 0,001 et 20 % en masse.

**7.** Un procédé selon la Revendication 6, dans lequel la matière grasse est présente dans une proportion allant jusqu'à 0,5 % en masse.

**8.** Un procédé selon l'une quelconque des Revendications précédentes, dans lequel la composition comprend en outre un ou plusieurs agent(s) tensioactif(s) sélectionnés parmi des agents tensioactifs non ioniques, amphotères et zwitterioniques.

**9.** Un procédé selon l'une quelconque des Revendications précédentes, dans lequel la composition comprend en outre un polymère de déposition.

**10.** Utilisation en tant qu'agent de conditionnement pour les cheveux d'un composé d'ammonium quaternaire lié par un ester représenté par la formule suivante :

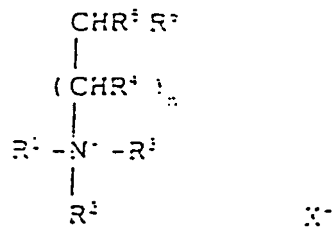

où n est un entier compris entre 1 et 8 ; $R^1$, $R^2$ et $R^3$ sont chacun indépendamment un groupe alkyle ou hydroxyalkyle comprenant 1 à 4 atome(s) de carbone ou un groupe benzyle ; chaque $R^4$ et chacun de $R^5$ et $R^6$ sont chacun indépendamment sélectionnés parmi -H, -OH,

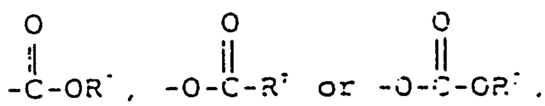

où $R^7$ est un groupe alkyle ou alkényle à chaîne longue linéaire ou ramifiée comprenant 8 à 28 atomes de carbone : et $X^-$ est un anion soluble dans l'eau ; sous réserve qu'au moins deux des groupes sélectionnés parmi $R^4$, $R^5$ et $R^6$ soient autres que -H ou -OH.

**11.** Une composition pour le soin des cheveux comprenant :

(i) un composé d'ammonium quaternaire lié par un ester représenté par la formule suivante :

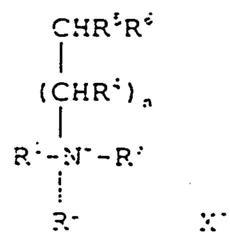

où n est un entier compris entre 1 et 8 ; $R^1$, $R^2$ et $R^3$ sont chacun indépendamment un groupe alkyle ou hydroxyalkyle comprenant 1 à 4 atome(s) de carbone ou un groupe benzyle ; chaque $R^4$ et chacun de $R^5$ et $R^6$ sont chacun indépendamment sélectionnés parmi -H, -OH,

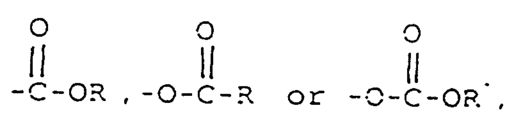

où $R^7$ est un groupe alkyle ou alkényle à chaîne longue linéaire ou ramifiée comprenant 8 à 28 atomes de carbone ; et $X^-$ est un anion soluble dans l'eau ; sous réserve qu'au moins deux des groupes sélectionnés parmi $R^4$, $R^5$ et $R^6$ soient autres que -H ou -OH ; et

(ii) environ 1 à 70 % en masse d'un polyglycoside d'alkyle en $C_5$-$C_{20}$.

**12.** Utilisation, dans le cadre de la préparation d'une composition destinée au conditionnement des cheveux d'un composé d'ammonium quaternaire lié par un ester représenté par la formule suivante :

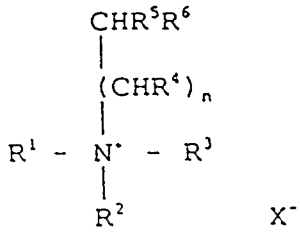

où n est un entier compris entre 1 et 8 ; $R^1$, $R^2$ et $R^3$ sont chacun indépendamment un groupe alkyle ou hydroxyalkyle comprenant 1 à 4 atome(s) de carbone ou un groupe benzyle ; chaque $R^4$ et chacun de $R^5$ et $R^6$ sont chacun indépendamment sélectionnés parmi -H, -OH,

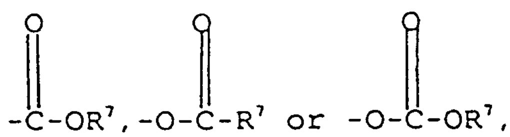
, , or ,

où $R^7$ est un groupe alkyle ou alkényle à chaîne longue linéaire ou ramifiée comprenant environ 8 à 28 atomes de carbone ; et $X^-$ est un anion soluble dans l'eau ; sous réserve qu'au moins deux des groupes sélectionnés parmi $R^4$, $R^5$ et $R^6$ soient autres que -H ou -OH.